(19) Europäisches Patentamt
European Patent Office
Office européen des brevets

(11) Publication number: 0 014 642
B1

(12) EUROPEAN PATENT SPECIFICATION

(45) Date of publication of patent specification: 21.03.84

(51) Int. Cl.[3]: A 61 K 9/06, A 61 K 31/41

(21) Application number: 80400154.3

(22) Date of filing: 31.01.80

(54) Ophthalmic compositions comprising combinations of carbonic anhydrase inhibitors and S-(-)-1-(tert-butylamino)-3-(4-morpholino-1,2,5-thiadiazol-3-yl)-oxy-2-propanol for topical application in the treatment of elevated intraocular pressure.

(30) Priority: 02.02.79 US 8903

(43) Date of publication of application:
20.08.80 Bulletin 80/17

(45) Publication of the grant of the patent:
21.03.84 Bulletin 84/12

(84) Designated Contracting States:
AT BE CH DE FR GB IT LU NL SE

(56) References cited:
FR - A - 2 281 757
FR - A - 2 332 008
GB - A - 860 371
GB - A - 1 524 405

UNLISTED DRUGS, vol. 29, no. 11, November 1977, Chatham, New York, U.S.A. "Moducren" 181r
CHEMICAL ABSTRACTS, vol. 89, no. 25, 18th December 1978, page 58, no. 209242n Columbus, Ohio, U.S.A. M.E. YABLONSKI et al.: "A fluorophotometric study of the effect of topical timolol on aqueous humor dynamics"

(73) Proprietor: MERCK & CO. INC.
126, East Lincoln Avenue P.O. Box 2000
Rahway New Jersey 07065 (US)

(72) Inventor: Smith, Robert L.
1355 Pickwick Lane
Lansdale, Pennsylvania 19446 (US)
Inventor: Hirschmann, Ralph F.
740 Palmer Place
Blue Bell, Pennsylvania 19422 (US)

(74) Representative: Corre, Jacques Denis Paul et al,
Cabinet Regimbeau 26, Avenue Kléber
F-75116 Paris (FR)

EP 0 014 642 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Ophthalmic compositions comprising combinations of carbonic anhydrase inhibitors and S-(—)-1-(tert-butylamino)-3-(4-morpholino-1,2,5-thiadiazol-3-yl)-oxy-2-propanol for topical application in the treatment of elevated intraocular pressure

## Disclosure of the Invention

This invention relates to compositions comprising carbonic anhydrase inhibitors and S-(—)-1-(*tert*-butylamino)-3-[(4-morpholino-1,2,5-thiadiazol-3-yl)-oxy]-2-propanol or its racemic modification that are topically effective in the treatment of elevated intraocular pressure. More particularly, it relates to compositions comprising a combination of S-(—)-1-(*tert*-butylamino)-3-[(4-morpholino-1,2,5-thiadiazol-3-yl)-oxy]-2-propanol or its racemic modification and an alkali metal salt of a salt-forming carbonic anhydrase inhibitor that, when applied topically to the eye, is transported to the target ocular tissue(s). In particular, this invention relates to a combination of S-(—)-1-(*tert*-butylamino)-3-[(4-morpholino-1,2,5-thiadiazol-3-yl)-oxy]-2-propanol or its racemic modification and a sodium or potassium salt of a salt-forming carbonic anhydrase inhibitor and the use thereof to lower intraocular pressure, especially in the treatment of ocular hypertension and glaucoma. It should be noted that, throughout this invention, the S-(—)-1-(*tert*-butylamino)-3-[(4-morpholino-1,2,5-thiadiazol-3-yl)-oxy]-2-propanol can be replaced by its racemic modification. Glaucoma is a degenerative disease of the eye wherein the pressure in the eye (i.e. introacular pressure) is too high for the normal function of the eye and as a result damage occurs to the optic nerve head and results in irreversible loss of visual function. If untreated, glaucoma will eventually lead to blindness. Ocular hypertension, i.e., the condition of elevated intraocular pressure without optic nerve head damage or characteristic glaucomatous visual field loss, is now believed by the majority of ophthalmologists to merely represent the earliest phase in the onset of glaucoma.

A number of the drugs presently employed to treat glaucoma are not entirely satisfactory, particularly in the earliest course of the disease when the side effects they produce are often worse than the symptoms of the disease.

A patient who is in the very early stages of the onset of glaucoma and is experiencing no overt discomfort symptoms will even, with high motivation, tend to neglect the regular administration of medication. At this stage, no pathogen has as yet developed and only elevated intraocular pressure is present. Thus, ocular hypertension may be present for an extended period prior to reaching the stage of pathological damage.

Pilocarpine, for example, although systemically harmless and quite effective, causes considerable local difficulties. The pupil constricts so that little light becomes available to the eye and the eye loses its ability to adapt from light to dark. Accommodation is stimulated so that the patient's refraction is sometimes incorrect and vision is blurred. The drug itself causes a local vasodilitation and red eyes and irritation are common. In short, although valuable, it really is unsatisfactory as a first line drug.

When carbonic anhydrase inhibitors are used systemically they have a number of disadvantages. While extremely effective in lowering intraocular pressure, they often cause a numbness and tingling, gastrointestinal upsets and, frequently, depression, lethargy, and a loss of appetite, and general malaise. These, in addition to the occasional more severe systemic complications such as aplastic anemia, are so common that many physicians are reluctant to routinely prescribe carbonic anhydrase inhibitors. As a consequence, only highly motivated patients who understand the seriousness of their condition will faithfully continue medication.

Reports in the literature indicate that carbonic anhydrase inhbitors are inactive topically. Even the extreme measure of direct injection into the anterior chamber is reported to have no pressure lowering effect.

Because carbonic anhydrase inhibitors have a profound effect in altering basic metabolism, the avoidance of a systemic route of administration serves to diminish, if not entirely eliminate, those side effects caused by metabolic acidosis such as vomiting, numbness, tingling, and general malaise and the like.

When combined with S-(—)-1-(*tert*-butylamino)-3-[(4-morpholino-1,2,5-thiadiazol-3-yl)-oxy]-2-propanol, there is experienced an additive effect that reduces the intraocular pressure below that obtained by either medicament individually. In addition, while there indeed may be a threshold beyond which intraocular pressure cannot be reduced, such a threshold is not evident from the combined use of the medicaments taught herein.

Furthermore, there exists a patient population who will benefit from a combination where the minimal dosage of each medicament is employed. Because of the additive effect, these patients can obtain a marked beneficial reduction in intraocular pressure on such a combination.

In the practice of this invention, the alkali metal salts of carbonic anhydrase inhibitors are prepared from the known, salt-forming carbonic anhydrase inhibitors, the following of which are preferred:

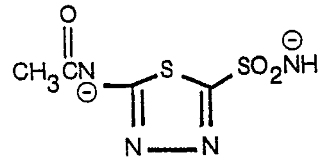

. $2M^{\oplus}$ ;

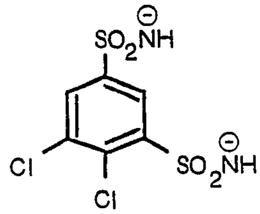

. $2M^{\oplus}$ ;

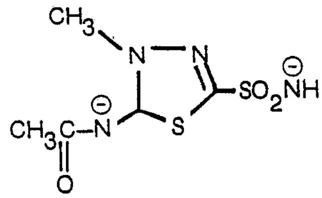

. $2M^{\oplus}$ ; and

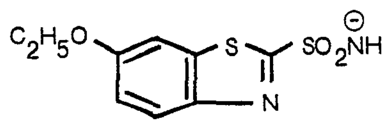

. $M^{\oplus}$

where $M^{\oplus}$ is an alkali metal cation. As used herein, the term "alkali metal" includes lithium, sodium, potassium, rubidium and cesium.

These salts may be hydroscopic and, as a consequence, may occur as hydrated species. However, when used in aqueous isotonic opthalmic solutions, hygroscopicity is obviously no longer a problem. When formulating into an insert, the material should be kept as dry as possible during manufacture and should be protected from excessive moisture during storage and before use. The preferred alkali metal salts include as cations, potassium, sodium and rubidium, with the most preferred being potassium and sodium. In both ophthalmic solutions and inserts, the potassium and sodium cations are preferred.

Other carbonic anhydrase inhibitors can be used if they are capable of forming an alkali metal salt. Especially useful are those carbonic anhydrase inhibitors which have the sulfamoyl moiety (i.e. $—SO_2NH_2$) as a substituent. This moiety can form the salt, $—SO_2\overset{\ominus}{N}HM^{\oplus}$, where $M^{\oplus}$ is defined as above. Thus, carbonic anhydrase inhibitors such as *p*-sulfamoylbenzoic acid, N-[5-(aminosulfonyl)-1,3,4-thiadiazol-2-yl]-propanamide, N-[5-(aminosulfonyl)-1,3,4-thiadiazol-2-yl]-butanamide and 5-benzenesulfonamido-1,3,4-thiadiazol-2-sulfonamide can also be used when formulating the carbonic anhydrase inhibitor alkali metal salts into an ophthalmic preparation.

When used alone to lower intraocular pressure, S-(—)-1-(*tert*-butylamino)-3-[(4-morpholino-1,2,5-thiadiazol-3-yl)-oxy]-2-propanol and its racemic modification are administered topically in solution, ointment or insert form as a pharmaceutically acceptable acid addition salt such as that derived from maleic acid and the like. However, in the present invention, the combination of a pharamaceutically acceptable acid addition salt of S-(—)-1-(*tert*-butylamino)-3-[(4-morpholino-1,2,5-thiadiazol-3-yl)-oxy]-2-propanol and its racemate with an alkali metal salt of a salt-forming carbonic anhydrase inhibitor is precluded by their physicochemical incompatibility, i.e., their admixture would result in formation of the topically-ineffective, parent carbonic anhydrase inhibitor and, thereby, reduce the therapeutic efficacy of the combined medicament. Hence, the present invention, requires that the parent drug, S-(—)-1-(*tert*-butylamino)-3-[(4-morpholino-1,2,5-thiadiazol-3-yl)-oxy]-2-propanol or its racemic modification, and *not* a pharamaceutically acceptable acid addition salt thereof, be combined with an alkali metal salt of a salt-forming carbonic anhydrase inhibitor.

In the combined ophthalmic dosage, from 0.1% to 5% by weight of the carbonic anhydrase inhibitor can be employed, together with from 0.01% to 5% by weight of S-(—)-1-(*tert*-butylamino)-3-[(4-morpholino-1,2,5-thiadiazol-3-yl)-oxy]-2-propanol. The objective is to administer a dose of from 0.001 to 10 mg. per eye per day to the patient.

Thus, in a liquid vehicle from 0.1% to 15% is a combination of S-(—)-1-(*tert*-butylamino)-3-[(4-morpholino-1,2,5-thiadiazol-3-yl)-oxy]-2-propanol and a carbonic anhydrase inhibitor salt, the remainder being carrier.

The combinations of this invention are preferably administered in the form of ophthalmic pharmaceutical compositions adapted for topical administration to the eye such as solutions, ointments or as solid inserts. Formulations of these compounds may contain from 0.01 to 5% and especially 0.5 to 2% of medicament. Higher dosages as, for example, about 10%, or lower dosages can be employed provided the dose is effective in lowering intraocular pressure. As a unit dosage from between 0.001 to 10.0 mg., preferably .005 to 2.0 mg., and especially 0.1 to 1.0 mg. of combined drugs is generally applied to the human eye, generally on a daily basis.

In the combination, the S-(—)-1-(*tert*-butylamino)-3-[(4-morpholino-1,2,5-thiadiazol-3-yl)-oxy]-2-propanol can comprise from 0.01% to 5% and especially 0.5% to 2% by weight of the combination dosage form. Higher dosages as, for example, about 10% or lower dosages can be employed provided the dose is effective in lowering intraocular pressure. As a unit dosage form between 0.001 to 5.0 mg., preferably .005 to 2.0 mg., and especially 0.005 to 1.0 mg. of the S-(—)-1-(*tert*-butylamino)-3-[(4-morpholino-1,2,5-thiadiazol-3-yl)-oxy]-2-propanol is generally applied to the human eye in the combined medication.

The pharmaceutical preparation which contains the compound may be conveniently admixed with a non-toxic pharmaceutical organic carrier, or with a pharmaceutically acceptable inorganic carrier. Typical of such pharmaceutically acceptable carriers are, for example, water, mixtures of water and watermiscible solvents such as lower alkanols or vegetable oils, polyalkylene glycols, petroleum based jelly, hydroxyethyl cellulose, ethyl oleat, carboxymethyl cellulose, polyvinylpyrrolidone, and other conventionally employed acceptable carriers. The pharmaceutical preparation may also contain non-toxic auxiliary substances such as emulsifying, preserving, wetting, bodying agents and the like, as for example, polyethylene glycols 200, 300, 400 and 600; carbowaxes 1,000, 1,500, 4,000, 6,000 and 10,000; antibacterial components such as quaternary ammonium compounds; phenylmercuric salts known to have cold sterilizing properties and which are non-injurious in use; thimerosal, methyl and propyl paraben, benzyl alcohol, phenyl ethanol; buffering ingredients such as alkali metal chloride, borate, acetate, gluconate buffers, and other conventional ingredients such as sorbitan monolaurate, triethanolamine, oleate, polyoxyethylene sorbitan monopalmitylate, dioctyl alkali metal sulfosuccinate, monothioglycerol, ethylenediamine tetraacetic acid and the like. When buffers are used that comprise an alkali metal cation, it is most highly preferred that the alkali metal cation of the buffer is identical to the alkali metal cation of the carbonic anhydrase inhibitor salt.

Additionally, suitable ophthalmic vehicles can be used as carrier media for the present purpose including conventional phosphate buffer vehicle systems, isotonic boric acid vehicles, isotonic alkali chloride vehicles, Tris and the like.

The pharmaceutical preparation may also be in the form of a solid insert. For example, one may use a solid, water soluble polymer as the carrier for the medicament. Inserts that are known in the art that are suitable for use with this combination include those set forth and described in U.S.P. 3,993,071; U.S.P. 3,986,510; U.S.P. 3,868,445; and U.S.P. 3,867,510 employing the formulation and fabrication techniques described therein. The polymer used to form the insert may be any water soluble non-toxic polymer, for example, cellulose derivatives such as methyl cellulose, alkali metal carboxymethyl cellulose, hydroxyethyl cellulose, hydroxypropyl cellulose, hydroxypropylmethyl cellulose; acrylates such as polyacrylic acid salts, ethyl acrylates and polyacrylamides; natural products such as gelatin, alginates, pectins, tragacanth, karaya, chondrus, agar, acacia; the starch derivatives such as starch acetate, hydroxyethyl starch ethers, hydroxypropyl starch, as well as other synthetic derivatives such as polyvinyl alcohol, polyvinyl pyrrolidone, polyvinyl methyl ether, polyethylene oxide, neutralized carbopol and xanthan gum and mixtures of said polymer.

If a solid insert is employed, it preferably is prepared from cellulose derivatives such as methyl cellulose, hydroxyethyl cellulose, hydroxypropyl cellulose or hydroxypropylmethyl cellulose or from other synthetic materials such as polyvinyl alcohol, polyvinyl pyrrolidone, polyethylene oxide or polyvinyl methylether.

Hydroxypropyl cellulose, one of the preferred polymers for the preparation of the insert is available in several polymeric forms, all of which are suitable in the preparation of these inserts. Thus, the products sold by Hercules, Inc. of Wilmington, Delaware, under the name KLUCEL such as KLUCEL HF, HWF, MF, GF, JF, LF and EF, which are intended for food or pharmaceutical use, are particularly useful. The molecular weight of these polymers useful for the purposes described herein may be at least 30,000 to about 1,000,000 or more.

Similarly, an ethylene oxide polymer having a molecular weight of up to 5,000,000 or greater, and preferably 100,000 to 5,000,000 can be employed.

Further, for example, POLYOX, a polymer supplied by Union Carbide Co., may be used having a molecular weight of about 50,000 to 5,000,000 or more and preferably 3,000,000 to 4,000,000. Other specific polymers which are useful are polyvinyl pyrrolidine having a molecular weight of from about 10,000 to about 1,000,000 or more, preferably up to about 350,000 and especially about 20,000 to 60,000; polyvinyl alcohol having a molecular weight of from about 30,000 to 1,000,000 or more, particularly, about 400,000 and, especially, from about 100,000 to about 200,000; hydroxypropylmethyl cellulose having a molecular weight of from about 10,000 to 1,000,000 or more particularly, up to about 200,000 and, especially, about 80,000 to about 125,000; methyl cellulose having a molecular weight of from about 10,000 to about 1,000,000 or more, preferably up to about 200,000 and, especially, about 50,000 to 100,000; and CARBOPOL (carboxyvinyl polymer) of B.F. Goodrich and Co., designated as grades 934, 940 and 941.

For the purpose of this invention the type and molecular weight of the polymer are not critical. Any water soluble polymers can be used which have an average molecular weight which will afford dissolution of the polymer and, accordingly, the medicament in any desired length of time. The inserts, therefore, can be prepared to allow for retention and, accordingly, effectiveness in the eye for any desired period. The insert can be in the form of a square, rectangle, oval, circle, doughnut, semi-circle, 1/4 moon shape, and the like. Preferably, the insert is in the form of a rod, doughnut, oval or 1/4 moon. The insert can be readily prepared, for example, by dissolving the medicament and the polymer in a suitable solvent and evaporating the resulting solution to afford a thin film of the polymer which can then be subdivided to prepare inserts of appropriate size. Alternatively, the insert can be prepared by intimately admixing polymer and the medicament and thereafter molding the resulting mixture under the influence of heat and pressure to form a thin film. Preferably, the inserts are prepared by molding or extrusion procedures well known in the art. The molded or extruded product can then be subdivided to afford inserts of suitable size for administration in the eye.

The insert can be of any suitable size which readily fits into the eye. For example, castings or compression molded films having a thickness of about 0.25 mm. to 1.50 mm. can be subdivided to obtain suitable inserts. Rectangular segments of the cast or compressed film having a thickness between about 0.5 and 1.5 mm. can be cut to afford shapes such as rectangular plates of 4 x 5—20 mm. or ovals of comparable size. Similarly, extruded rods having a diameter between about 0.5 and 1.5 mm. can be cut into suitable sections to provide the desired amount of polymer. For example, rods of 1.0 to 1.5 mm. in diameter and about 2—20 mm. long are found to be satisfactory. The inserts may also be directly formed by injection molding. It is preferred that the ophthalmic inserts containing the medicament of the present invention be formed so that they are smooth and do not have any sharp edges or corners which could cause damage to the eye. Since the terms smooth and sharp edges or corners are subjective terms, in this application these terms are used to indicate that excessive irritation of the eye will not result from the use of the insert.

The medicated ocular inserts can also contain plasticizers, buffering agents, appropriate inert fillers, and preservatives. Plasticizers suitable for this purpose must, of course, also be completely soluble in the lacrimal fluids of the eye. Examples of suitable plasticizers that might be mentioned are water, polyethylene glycol, propylene glycol, glycerine, trimethylol propane, di- and tripropylene glycol, hydroxypropyl sucrose and the like. Typically, such plasticizers can be present in the medicated ophthalmic insert in an amount ranging from 1 up to about 30% by weight. A particularly preferred plasticizer is water which is present in amounts of at least about 5% up to about 40%. In actual practice, a water content of from about 10% to about 20% is preferred since it may be easily accomplished and adds the desired softness and pliability to the insert.

When plasticizing the solid medicinal product with water, the product is contacted with air having a relative humidity of at least 40% until said product picks up at least about 5% water and becomes softer and more pliable. In a preferred embodiment, the relative humidity of the air is from about 60% to about 99% and the contact is continued until the water is present in the product in amounts of from about 10% to about 20%.

Suitable water soluble preservatives which may be employed in the insert are alkali bisulfate, alkali thiosulfate, ascorbate, benzalkonium chloride, chlorobutanol, thimerosal, phenylmercuric acetate, phenylmercuric borate, parabens, benzyl alcohol and phenyl ethanol. These agents may be present in amounts of from 0.001 to 5% by weight of solid insert, and preferably 0.1 to 2%.

Suitable water soluble buffering agents are alkali, alkali earth carbonates, phosphates, bicarbonates, citrates, borates, and the like, such as sodium or potassium phosphate, citrate, borate, acetate, bicarbonate and carbonate. These agents may be present in amounts sufficient to obtain a pH of the system of between 5.5 to 8.1 and especially 7—8; usually up to about 2% by weight of polymer. The insert may contain from about 1 mg. to 100 mg. of water soluble polymer, more particularly from 5 to 50 mg. and especially from 5 to 20 mg. The medicament is present from about 0.1 to about 25% by weight of insert.

The following examples serve to more fully illustrate this invention.

Example 1

| Solution Composition | a | b |
|---|---|---|
| Na⊕ salt of methazolamide | 1 mg. | 15 mg. |
| S-(—)-1-(*tert*-butylamino)-3-[(4-morpholino-1,2,5-thiadiazol-3-yl)-oxy]-2-propanol | 0.1 mg. | 1.0 mg. |
| Benzalkonium chloride | 0.10 mg. | 0.10 mg. |
| Water for injection q.s. ad. | 1.0 ml. | 1.0 ml. |

phosphate buffer salts (may be added to further adjust the pH to the level desired) and benzalkonium chloride are added to and dissolved in water. The solution is rendered sterile by filtration through a sterilizing filter.

Example 2

| | |
|---|---|
| Na⊕ salt of methazolamide | 5 mg. |
| S-(—)-1-(*tert*-butylamino)-3-[(4-morpholino-1,2,5-thiadiazol-3-yl)-oxy]-2-propanol | 0.5 mg. |
| Petrolatum q.s. ad. | 1 gram |

and the petrolatum are aseptically combined.

Example 3

| | |
|---|---|
| Na⊕ salt of methazolamide | 1 mg. |
| S-(—)-1-(*tert*-butylamino)-3-[(4-morpholino-1,2,5-thiadiazol-3-yl)-oxy]-2-propanol | 0.1 mg. |
| Hydroxypropylmethyl cellulose q.s. | 12 mg. |

Ophthalmic inserts are manufactured from a solvent cast film prepared by making a viscous solution of the powder blend using a methanol/water solvent system (10 ml. methanol is added to 2.5 g. of powder blend, to which 11 ml. of water (in three divided portions) is added). The solution is placed on a Teflon polytetrafluoroethylene plate and allowed to dry at ambient conditions. After drying, the film is placed in an 88% relative humidity cabinet until it is pliable. Appropriately sized inserts are then cut from the film.

Example 4

| | |
|---|---|
| Na⊕ salt of methazolamide | 1 mg. |
| S-(—)-1-(*tert*-butylamino)-3-[(4-morpholino-1,2,5-thiadiazol-3-yl)-oxy]-2-propanol | 0.1 mg. |
| Hydroxypropyl cellulose q.s. a.d. | 12 mg. |

Opthalmic inserts are manufactured from compression molded films which are prepared on a Carver Press by subjecting the powder mixture of the above ingredients to a compressional force of

12,000 lbs. (gauge), at 350°F for one minute. The film is cooled under pressure by having cold water circulate in the platen. Ophthalmic inserts are then individually cut from the film with a punch. Each insert is placed into a vial, which is then placed in a humidity cabinet (88% relative humidity at 30°C) for two to four days. After removal from the humidity cabinet, the vials are stoppered and then capped. The vials containing the hydrated insert are then sterilized by means known in the art such as irradiation with high energy electron beams, gamma radiation with a suitable radiation source such as $Co^{60}$.

Example 5

Preparation of N-[5-(aminosulfonyl)-3-methyl-1,3,4-thiadiazol-2(3H)-ylidene]-acetamide Potassium Salt

To a solution of 0.1*N* potassium hydroxide in methanol (1.19 l., 0.119 mole) is added distilled water (300 ml.) and N-[5-(aminosulfonyl)-3-methyl-1,3,4-thiadiazol-2(3H)-ylidene]-acetamide (28.12 g., 0.119 mole) at room temperature. The resulting mixture is swirled until complete dissolution of the suspended solid occurs, whereupon, the clear solution is evaporated *in vacuo* at 40°C leaving a colorless residual solid. The solid is dissolved in distilled water (300 ml.) providing a clear solution which is evaporated *in vacuo* at 40°C. The residual solid is collected and dried over phosphorous pentoxide at 100°C *in vacuo* for 16 hours to afford analytically pure N-[5-(aminosulfonyl)-3-methyl-1,3,4-thiadiazol-2(3H)-ylidene]-acetamide potassium salt as a colorless solid (30.2 g., 92.6%), m.p. 260—261°C with dec.

Example 6

Preparation of N-[5-(aminosulfonyl)-3-methyl-1,3,4-thiadiazol-2(3H)-ylidene]-acetamide Sodium Salt

To 0.1*N* sodium hydroxide (120 ml., 0.12 mole) is added N-[5-(aminosulfonyl)-3-methyl-1,3,4-thiadiazol-2(3H)-ylidene]-acetamide (28.32 g., 0.12 mole) with stirring at room temperature. Stirring is continued at room temperature until a solution is obtained. The resulting solution is diluted with distilled water (120 ml.) and filtered. Evaporation of the clear filtrate *in vacuo* at 40°C provides a colorless solid residue which is dried *in vacuo* at 80°C for 16 hours, collected and re-dried *in vacuo* over phosphorous pentoxide at 100°C for four hours to afford analytically pure N-[5-(aminosulfonyl)-3-methyl-1,3,4-thiadiazol-2(3H)-ylidene]-acetamide sodium salt as a colorless solid (28.75 g., 92.8%), m.p. 230—235°C.

Prepared analogously using equivalent molar quantities of reactants are other carbonic anhydrase inhibitor alkali metal salts such as those illustrated in Table I below.

TABLE I

Carbonic Anhydrase Inhibitor Salts

| Compound | m.p. (°C With Dec.) |
|---|---|
| 4,5-Dichloro-m-benzenedisulfonamide dipotassium salt . hemihydrate | 215—220 |
| 4,5-Dichloro-m-benzenedisulfonamide disodium salt . hemihydrate | 300 |
| 4,5-Dichloro-m-benzenedisulfonamide dirubidium salt | 260 |
| N-[5-(aminosulfonyl)-1,3,4-thiadiazol-2-yl]-acetamide dipotassium salt | 220 |
| 218—222 thiadiazol-2-yl]-acetamide disodium salt . 0.25 hydrate | |

It has been noted that the sodium salts of carbonic anhydrase inhibitors in combination with S-(—)-1-(*tert*-butylamino)-3-[(4-morpholino-1,2,5-thiadiazol-3-yl)-oxy]-2-propanol when administered in the form of inserts, as opposed to liquid formulation administered by means of drops into the eye, display a more rapid onset of ocular hypotensive activity than the potassium salts of carbonic anhydrase inhibitors. However, these two salt forms (e.g., sodium and potassium), achieve comparable ceiling effects. Therefore, when rapid onset of action is desired it is preferred to employ the sodium salt when inserts are used as the carrier or vehicle.

Generally, administration of one to two drops per eye of the liquid formulation from twice a day to once every 8 hours is sufficient to control ocular hypertension. When using inserts to administer the medication, one insert per day per eye is suitable. However, as with all medication, medical monitoring of symptoms must be employed to enable one to arrive at the optimum dosage for the patient.

**Claims for the Contracting States: BE CH DE FR GB IT LU NL SE**

1. An ophthalmic composition for lowering intraocular pressure, characterized in that it comprises an intraocular pressure reducing amount of an alkali metal salt of a carbonic anhydrase inhibitor, and S-(—)-1-(*tert*-butylamino)-3-[(4-morpholino-1,2,5-thiadiazol-3-yl)-oxy]-2-propanol or its racemic modification together with an ophthalmologically acceptable carrier for topical application.

2. A composition according to claim 1 where the carbonic anhydrase inhibitor is selected from the group consisting of 4,5-dichloro-m-benzene-disulfonamide; N-[5-(aminosulfonyl)-1,3,4-thiadiazol-2-yl]-acetamide; N-[5-(aminosulfonyl)-3-methyl-1,3,4-thiadiazol-2(3H)-ylidene]-acetamide; *p*-sulfamoylbenzoic acid; N-[5-(aminosulfonyl)-1,3,4-thiadiazol-2-yl]-propanamide; N-[5-(aminosulfonyl)-1,3,4-thiadiazol-2-yl]-butanamide; 5-benzenesulfonamido-1,3,4-thiadiazol-2-sulfonamide and 6-ethoxy-2-benzothiazolesulfonamide.

3. A composition according to anyone of claims 1 and 2 where the alkali metal salt contains the $—SO_2\overset{\ominus}{N}HM^{\oplus}$ moiety(ies), wherein $M^{\oplus}$ is an alkali metal ion, a salt of the functional group(s) $—SO_2NH_2$.

4. A composition according to anyone of claims 1 to 3 where the salt is a potassium salt.

5. A composition according to anyone of claims 1 to 3 where the salt is a sodium salt.

6. A composition according to anyone of claims 1 to 3 where the salt is a rubidium salt.

7. A composition according to anyone of claims 1 to 6 where the alkali metal salt of the carbonic anhydrase inhibitor is from 0.1 to 15% by weight of said composition.

8. A composition according to anyone of claims 1 to 7 where the carrier is an isotonic aqueous solution.

9. A composition according to anyone of claims 1 to 8 where there is included in the composition a preservative so said composition is bacteriostatic.

10. A composition according to anyone of claims 1 to 9 where the carrier comprises a solid water soluble polymer

11. A composition according to claim 10 where the carrier is a cellulose gum.

12. An ophthalmologically acceptable solid water soluble polymeric insert comprising an intraocular pressure lowering amount of a potassium or sodium salt of a carbonic anhydrase inhibitor selected from the group consisting of 4,5-dichloro-m-benzene-disulfonamide; N-[5-(aminosulfonyl)-1,3,4-thiadiazol-2-yl]-acetamide; N-[5-(aminosulfonyl)-3-methyl-1,3,4-thiadiazol-2(3H)-ylidene]-acetamide; *p*-sulfamoylbenzoic acid; N-[5-(aminosulfonyl)-1,3,4-thiadiazol-2-yl]-propanamide; N-[5-(aminosulfonyl)-1,3,4-thiadiazol-2-yl]-butanamide, 5-benzenesulfonamido-1,3,4-thiadiazol-2-sulfonamide and 6-ethoxy-2-benzothiazolesulfonamide and an appropriate amount of S-(—)-1-(tert-butylamino)-3-[(4-morpholino-1,2,5-thiadiazol-3-yl)-oxy]-2-propanol or its racemic modification.

13. An insert according to claim 12 where the polymeric carrier is a polymer soluble in lacrimal fluids.

14. An insert according to anyone of claims 12 and 13 where the salt is the potassium salt.

15. An insert according to anyone of claims 12 and 13 where the salt is the sodium salt.

**Claims for the Contracting State: AT**

1. Process for preparing an ophthalmic composition for lowering intraocular pressure, characterized in that it comprises mixing an intraocular pressure reducing amount of an alkali metal salt of a carbonic anhydrase inhibitor, and S-(—)-1-(*tert*-butylamino)-3-[(4-morpholino-1,2,5-thiadiazol-3-yl)-oxy]-2-propanol or its racemic modification together with an ophthalmologically acceptable carrier for topical application.

2. A process according to claim 1 where the carbonic anhydrase inhibitor is selected from the group consisting of 4,5-dichloro-m-benzene-disulfonamide; N-[5-(aminosulfonyl)-1,3,4-thiadiazol-2-yl]-acetamide; N-[5-(aminosulfonyl)-3-methyl-1,3,4-thiadiazol-2(3H)-ylidene]-acetamide; *p*-sulfamoylbenzoic acid; N-[5-(aminosulfonyl)-1,3,4-thiadiazol-2-yl]-propanamide; N-[5-(aminosulfonyl)-1,3,4-thiadiazol-2-yl]-butanamide; 5-benzenesulfonamido-1,3,4-thiadiazol-2-sulfonamide and 6-ethoxy-2-benzothiazolesulfonamide.

3. A process according to anyone of claims 1 and 2 where the alkali metal salt contains the $—SO_2\overset{\ominus}{N}HM^{\oplus}$ moiety(ies), where $M^{\oplus}$ is an alkali metal ion, a salt of the functional group(s) $—SO_2NH_2$.

4. A process according to anyone of claims 1 to 3 where the salt is a potassium salt.

5. A process according to anyone of claims 1 to 3 where the salt is a sodium salt.

6. A process according to anyone of claims 1 to 3 where the salt is a rubidium salt.

7. A process according to anyone of claims 1 to 6 where the alkali metal salt of the carbonic anhydrase inhibitor is from 0.1 to 15% by weight of said composition.

8. A process according to anyone of claims 1 to 7 where the carrier is an isotonic aqueous solution.

9. A process according to anyone of claims 1 to 8 where there is included in the composition a preservative so said composition is bacteriostatic.

10. A process according to anyone of claims 1 to 9 where the carrier comprises a solid water soluble polymer

11. A process according to claim 10 where the carrier is a cellulose gum.

12. A process for preparing an ophthalmologically acceptable solid water soluble polymeric insert comprising mixing an intraocular pressure lowering amount of a potassium or sodium salt of a carbonic anhydrase inhibitor selected from the group consisting of 4,5-dichloro-m-benzene-disulfonamide; N-[5-(aminosulfonyl)-1,3,4-thiadiazol-2-yl]-acet-amide; N-[5-(aminosulfonyl)-3-methyl-1,3,4-thiadiazol-2(3H)-ylidene]-acetamide; *p*-sulfamoylbenzoic acid; N-[5-(aminosulfonyl)-1,3,4-thiadiazol-2-yl]-propanamide; N-[5-(aminosulfonyl)-1,3,4-thiadiazol-2-yl]-butanamide, 5-benzenesulfonamido-1,3,4-thiadiazol-2-sulfonamide and 6-ethoxy-2-benzothiazolesulfonamide and an appropriate amount of S-(—)-1-(tert-butylamino)-3-[(4-morpholino-1,2,5-thiadiazol-3-yl)-oxy]-2-propanol or its racemic modification with a water soluble polymer and shaping the resulting mixture in the form of an insert.

13. A process according to claim 12 where the polymeric carrier is a polymer soluble in lacrimal fluids.

14. A process according to anyone of claims 12 and 13 where the salt is the potassium salt.

15. A process according to anyone of claims 12 and 13 where the salt is the sodium salt.

**Revendications pour les Etats contractants: BE CH DE FR GB IT LU NL SE**

1. Une composition ophtalmique pour abaisser la pression intra-oculaire caractérisée en ce qu'elle comprend une quantité abaissant la pression intra-oculaire d'un sel de métal alcalin d'un inhibiteur d'anhydrase carbonique et du S-(—)-1-(tertbutylamino)-3-[(4-morpholino-1,2,5-thiadiazol-3-yl)-oxy]-2-propanol ou sa modification racémique avec un véhicule acceptable en ophtalmologie pour l'application locale.

2. Une composition selon la revendication 1 dans laquelle l'inhibiteur d'anhydrase carbonique est choisi parmi le groupe constitué par:
. le 4,5-dichloro-m-benzènedisulfonamide;
. le N-[5-(aminosulfonyl)-1,3,4-thiadiazol-2-yl]-acétamide;
. le N-[5-(aminosulfonyl)-3-méthyl-1,3,4-thiadiazol-2(3H)-ylidène]-acétamide;
. l'acide p-sulfamoylbenzoïque;
. le N-[5-(aminosulfonyl)-1,3,4-thiadiazol-2-yl]-propanamide;
. le N-[5-aminosulfonyl)-1,3,4-thiadiazol-2-yl]-butanamide;
. le 5-benzènesulfonamido-1,3,4-thiadiazol-2-sulfonamide; et
. le 6-éthoxy-2-benzothiazolesulfonamide.

3. Une composition selon l'une quelconque des revendications 1 et 2, dans laquelle le sels de métal alcalin contient le(s) fragment(s) $—SO_2\overset{\ominus}{N}HM^{\oplus}$ où $M^{\oplus}$ est un ion de métal alcalin, un sel du (des) groupe(s) fonctionnel(s) $—SO_2NH_2$.

4. Une composition selon l'une quelconque des revendications 1 à 3 où le sel est un sel de potassium.

5. Une composition selon l'une quelconque des revendications 1 à 3 où le sel est un sel de sodium.

6. Une composition selon l'une quelconque des revendications 1 à 3 où le sel est un sel de rubidium.

7. Une composition selon l'une quelconque des revendications 1 à 6 où le sel de métal alcalin de l'inhibiteur d'anhydrase carbonique constitue 0,1 à 15% du poids de ladite composition.

8. Une composition selon l'une quelconque des revendications 1 à 7 où le véhicule est une solution aqueuse isotonique.

9. Une composition selon l'une quelconque des revendications 1 à 8, la composition comprenant un conservateur, si bien que ladite composition est bactériostatique.

10. Une composition selon l'une quelconque des revendications 1 à 9 où le véhicule comprend un polymère solide soluble dans l'eau.

11. Une composition selon la revendication 10 où le véhicule est une gomme de cellulose.

12. Un insert polymère soluble dans l'eau solide acceptable en ophtalmologie comprenant une quantité abaissant la pression intra-oculaire d'un sel de potassium ou de sodium d'un inhibiteur d'anhydrase carbonique choisi parmi le groupe constitué par:
. le 4,5-dichloro-m-benzènedisulfonamide;
. le N-[5-(aminosulfonyl)-1,3,4-thiadiazol-2-yl]-acétamide;

. le N-[5-(aminosulfonyl)-3-méthyl-1,3,4-thiadiazol-2(3H)-ylidène]-acétamide;
. l'acide p-sulfamoylbenzoïque;
. le N-[5-(aminosulfonyl)-1,3,4-thiadiazol-2-yl]-propanamide;
. le N-[5-(aminosulfonyl)-1,3,4-thiadiazol-2-yl]-butanamide;
. le 5-benzènesulfonamido-1,3,4-thiadiazol-2-sulfonamide; et
. le 6-éthoxy-2-benzothiazolesulfonamide

et une quantité appropriée de S-(—)-1-(tertbutylamino)-3-[(4-morpholino-1,2,5-thiadiazol-3-yl)-oxy]-2-propanol ou sa modification racémique.

13. Un insert selon la revendication 12 où le véhicule polymère est un polymère soluble dans les liquides lacrymaux.

14. Un insert selon l'une quelconque des revendications 12 et 13 où le sel est le sel de potassium.

15. Un insert selon l'une quelconque des revendications 12 et 13 où le sel est le sel de sodium.

**Revendications pour l'Etat contractant: AT**

1. Un procédé pour la préparation d'une composition ophtalmique pour abaisser la pression intra-oculaire caractérisé en ce que l'on mélange une quantité abaissant la pression intra-oculaire d'un sel de métal alcalin d'un inhibiteur d'anhydrase carbonique et du S-(—)-1-(tertbutylamino)-3-[(4-morpholino-1,2,5-thiadiazol-3-yl)-oxy]-2-propanol ou sa modification racémique avec un véhicule acceptable en ophtalmologie pour l'application locale.

2. Un procédé selon la revendication 1 dans lequel l'inhibiteur d'anhydrase carbonique est choisi parmi le groupe constitué par:
. le 4,5-dichloro-m-benzènedisulfonamide;
. le N-[5-(aminosulfonyl)-1,3,4-thiadiazol-2-yl]-acétamide;
. le N-[5-(aminosulfonyl)-3-méthyl-1,3,4-thiadiazol-2(3H)-ylidène]-acétamide;
. l'acide p-sulfamoylbenzoïque;
. le N-[5-(aminosulfonyl)-1,3,4-thiadiazol-2-yl]-propanamide;
. le N-[5-aminosulfonyl)-1,3,4-thiadiazol-2-yl]-butanamide;
. le 5-benzènesulfonamido-1,3,4-thiadiazol-2-sulfonamide; et
. le 6-éthoxy-2-benzothiazolesulfonamide.

3. Un procédé selon l'une quelconque des revendications 1 et 2, dans lequel le sels de métal alcalin contient le(s) fragment(s) $—SO_2\overset{\ominus}{N}HM^{\oplus}$ où $M^{\oplus}$ est un ion de métal alcalin, un sel du (des) groupe(s) fonctionnel(s) $—SO_2NH_2$.

4. Un procédé selon l'une quelconque des revendications 1 à 3 où le sel est un sel de potassium.

5. Un procédé selon l'une quelconque des revendications 1 à 3 où le sel est un sel de sodium.

6. Un procédé selon l'une quelconque des revendications 1 à 3 où le sel est un sel de rubidium.

7. Un procédé selon l'une quelconque des revendications 1 à 6 où le sel de métal alcalin de l'inhibiteur d'anhydrase carbonique constitue 0,1 à 15% du poids de ladite composition.

8. Un procédé selon l'une quelconque des revendications 1 à 7 où le véhicule est une solution aqueuse isotonique.

9. Un procédé selon l'une quelconque des revendications 1 à 8, la composition comprenant un conservateur, si bien que ladite composition est bactériostatique.

10. Un procédé selon l'une quelconque des revendications 1 à 9 où le véhicule comprend un polymère solide soluble dans l'eau.

11. Un procédé selon la revendication 10 où le véhicule est une gomme de cellulose.

12. Un procédé pour la préparation d'un insert polymère solide soluble dans l'eau acceptable en ophtalmologie caractérisé en ce que l'on mélange une quantité abaissant la pression intra-oculaire d'un sel de potassium ou de sodium d'un inhibiteur d'anhydrase carbonique choisi parmi le groupe constitué par:
. le 4,5-dichloro-m-benzènedisulfonamide;
. le N-[5-(aminosulfonyl)-1,3,4-thiadiazol-2-yl]-acétamide;
. le N-[5-(aminosulfonyl)-3-méthyl-1,3,4-thiadiazol-2(3H)-ylidène]-acétamide;
. l'acide p-sulfamoylbenzoïque;
. le N-[5-(aminosulfonyl)-1,3,4-thiadiazol-2-yl]-propanamide;
. le N-[5-(aminosulfonyl)-1,3,4-thiadiazol-2-yl]-butanamide;
. le 5-benzènesulfonamido-1,3,4-thiadiazol-2-sulfonamide; et
. le 6-éthoxy-2-benzothiazolesulfonamide

et une quantité appropriée de S-(—)-1-(tertbutylamino)-3-[(4-morpholino-1,2,5-thiadiazol-3-yl)-oxy]-2-propanol ou sa modification racémique avec un polymère soluble dans l'eau et en ce que l'on façonne le mélange obtenu sous forme d'un insert.

13. Un procédé selon la revendication 12 où le véhicule polymère est un polymère soluble dans les liquides lacrymaux.

14. Un procédé selon l'une quelconque des revendications 12 et 13 où le sel est le sel de potassium.

15. Un procédé selon l'une quelconque des revendications 12 et 13 où le sel de sodium.

**Patentansprüche für die Vertragsstaaten: BE CH DE FR GB IT LU NL SE**

1. Ophthalmische Zusammensetzung zur Verringerung des Intraokulardrucks, dadurch gekennzeichnet, daß sie eine de intraokularen Druck senkende Menge eines Alkalimetallsalzes eines Carboanhydrase-Inhibitors und S-(—)-1-(tert.-Butylamino)-3-[(4-morpholino-1,2,5-thiadiazol-3-yl)-oxy]-2-propanol oder seine racemische Modifikation zusammen mit einem ophthalmologisch brauchbaren Träger für die topische Verabreichung enthält.

2. Zusammensetzung nach Anspruch 1, in der der Carboanhydrase-Inhibitor ausgewählt ist aus der Gruppe von 4,5-Dichlor-m-benzoldisulfonamid; N-[5-(Aminosulfonyl)-1,3,4-thiadiazol-2-yl]-acetamid; N-[5-(Aminosulfonyl)-3-methyl-1,3,4-thiadiazol-2(3H)-yliden]-acetamid; p-Sulfamoylbenzoesäure; N-[5-(Aminosulfonyl)-1,3,4-thiadiazol-2-yl]-propanamid; N-[5-(Aminosulfonyl)-1,3,4-thiadiazol-2-yl]-butanamid; 5-Benzolsulfonamido-1,3,4-thiadiazol-2-sulfonamid und 6-Ethoxy-2-benzothiazolsulfonamid.

3. Zusammensetzung nach einem der Ansprüche 1 und 2, in dem das Alkalimetallsalz die Gruppe(n) $—SO_2\overset{\ominus}{N}HM^{\oplus}$, worin $M^{\oplus}$ ein Alkalimetallion darstellt, ein Salz der funktionellen Gruppe(n) $—SO_2NH_2$, enthält.

4. Zusammensetzung nach einem der Ansprüche 1 bis 3, worin das Salz ein Kaliumsalz ist.

5. Zusammensetzung nach einem der Ansprüche 1 bis 3, worin das Salz ein Natriumsalz ist.

6. Zusammensetzung nach einem der Ansprüche 1 bis 3, worin das Salz ein Rubidiumsalz ist.

7. Zusammensetzung nach einem der Ansprüche 1 bis 6, worin das Alkalimetallsalz des Carboanhydrase-Inhibitors 0,1 bis 15 Gewichtsprozent der Zusammensetzung beträgt.

8. Zusammensetzung nach einem der Ansprüche 1 bis 7, worin der Träger eine isotonische wäßrige Lösung ist.

9. Zusammensetzung nach einem der Ansprüche 1 bis 8, wobei in die Zusammensetzung ein Konservierungsmittel einbezogen ist, so daß die Zusammensetzung bakteriostatisch ist.

10. Zusammensetzung nach einem der Ansprüche 1 bis 9, in der der Träger ein festes wasserlösliches Polymeres umfaßt.

11. Zusammensetzung nach Anspruch 10, in der der Träger ein Cellulosegummi ist.

12. Ophthalmologisch brauchbare feste wasserlösliche polymere Einlage, enthaltend eine den intraokularen Druck senkende Menge eines Kalium- oder Natriumsalzes eines Carboanhydrase-Inhibitors, ausgewählt aus der Gruppe von 4,5-Dichlor-m-benzoldisulfonamid; N-[5-(Aminosulfonyl)-1,3,4-thiadiazol-2-yl]-acetamid; N-[5-(Aminosulfonyl)-3-methyl-1,3,4-thiadiazol-2(3H)-yliden]-acetamid; p-Sulfamoylbenzoesäure; N-[5-(Aminosulfonyl)-1,3,4-thiadiazol-2-yl]-propanamid; N-[5-(Aminosulfonyl)-1,3,4-thiadiazol-2-yl]-butanamid, 5-Benzolsulfonamido-1,3,4-thiadiazol-2-sulfonamid und 6-Ethoxy-2-benzothiazolsulfonamid und eine geeignete Menge von S-(—)-1-(tert.-Butylamino)-3-[(4-morpholino-1,2,5-thiadiazol-3-yl)-oxy]-2-propanol oder seiner racemischen Modifikation.

13. Einlage nach Anspruch 12, worin der polymere Träger ein in Tränenflüssigkeiten lösliches Polymeres ist.

14. Einlage nach einem der Ansprüche 12 und 13, worin das Salz das Kaliumsalz ist.

15. Einlage nach einem der Ansprüche 12 und 13, worin das Salz das Natriumsalz ist.

**Patentansprüche fur die Vertragsstaat: AT**

1. Verfahren zur Herstellung einer ophthalmischen Zusammensetzung zur Verringerung des Intraokulardrucks, dadurch gekennzeichnet, daß man eine den intraokularen Druck senkende Menge eines Alkalimetallsalzes eines Carboanhydrase-Inhibitors und S-(—)-1-(tert.-Butylamino)-3-[(4-morpholino-1,2,5-thiadiazol-3-yl)-oxy]-2-propanol oder seine racemische Modifikation zusammen mit einem ophthalmologisch brauchbaren Träger für die topische Verabreichung mischt.

2. Verfahren nach Anspruch 1, in dem der Carboanhydrase-Inhibitor ausgewählt ist aus der Gruppe von 4,5-Dichlor-m-benzoldisulfonamid; N-[5-(Aminosulfonyl)-1,3,4-thiadiazol-2-yl]-acetamid; N-[5-(Aminosulfonyl)-3-methyl-1,3,4-thiadiazol-2(3H)-yliden]-acetamid; p-Sulfamoylbenzoesäure; N-[5-(Aminosulfonyl)-1,3,4-thiadiazol-2-yl]-propanamid; N-[5-(Aminosulfonyl)-1,3,4-thiadiazol-2-yl]-butanamid; 5-Benzolsulfonamido-1,3,4-thiadiazol-2-sulfonamid und 6-Ethoxy-2-benzothiazolsulfonamid.

3. Verfahren nach einem der Ansprüche 1 und 2, in dem das Alkalimetallsalz die Gruppe(n) $—SO_2\overset{\ominus}{N}HM^{\oplus}$, worin $M^{\oplus}$ ein Alkalimetallion darstellt, ein Salz der funktionellen Gruppe(n) $—SO_2NH_2$, enthält.

4. Verfahren nach einem der Ansprüche 1 bis 3, worin das Salz ein Kaliumsalz ist.

5. Verfahren nach einem der Ansprüche 1 bis 3, worin das Salz ein Natriumsalz ist.

6. Verfahren nach einem der Ansprüche 1 bis 3, worin das Salz ein Rubidiumsalz ist.

7. Verfahren nach einem der Ansprüche 1 bis 6, worin das Alkalimetallsalz des Carboanhydrase-Inhibitors 0,1 bis 15 Gewichtsprozent der Zusammensetzung beträgt.

8. Verfahren nach einem der Ansprüche 1 bis 7, worin der Träger eine isotonische wäßrige Lösung ist.

9. Verfahren nach einem der Ansprüche 1 bis 8, wobei in die Zusammensetzung ein Konservierungsmittel einbezogen ist, so daß die Zusammensetzung bakteriostatisch ist.

10. Verfahren nach einem der Ansprüche 1 bis 9, in dem der Träger ein festes wasserlösliches Polymeres umfaßt.

11. Verfahren nach Anspruch 10, in dem der Träger ein Cellulosegummi ist.

12. Verfahren zur Herstellung einer ophthalmologischen brauchbaren festen wasserlöslichen polymeren Einlage, enthaltend Mischung einer den intraokularen Druck senkenden Menge eines Kalium- oder Natriumsalzes eines Carboanhydrase-Inhibitors, ausgewählt aus der Gruppe von 4,5-Dichlor-m-benzoldisulfonamid; N-[5-(Aminosulfonyl)-1,3,4-thiadiazol-2-yl]-acetamid; N-[5-(Aminosulfonyl)-3-methyl-1,3,4-thiadiazol-2(3H)-yliden]-acetamid; p-Sulfamoylbenzoesäure; N-[5-(Aminosulfonyl)-1,3,4-thiadiazol-2-yl]-propanamid; N-[5-(Aminosulfonyl)-1,3,4-thiadiazol-2-yl]-butanamid, 5-Benzolsulfonamido-1,3,4-thiadiazol-2-sulfonamid und 6-Ethoxy-2-benzothiazolsulfonamid und einer geeignete Menge von S-(—)-1-(tert.-Butylamino)-3-[(4-morpholino-1,2,5-thiadiazol-3-yl)-oxy]-2-propanol oder seiner racemischen Modifikation zusammen mit einem wasserloeslichen Polymer, und Verformung der so erhaltenen Mischung in Form einer Einlage.

13. Verfahren nach Anspruch 12, worin der polymere Träger ein in Tränenflüssigkeiten lösliches Polymeres ist.

14. Verfahren nach einem der Ansprüche 12 und 13, worin das Salz das Kaliumsalz ist.

15. Verfahren nach einem der Ansprüche 12 und 13, worin das Salz das Natriumsalz ist.